# EUROPEAN PATENT APPLICATION

(11) **EP 4 641 171 A1**
(43) Date of publication of application: **29.10.2025**
(21) Application number: 24172059.8
(22) Date of filing: 24.04.2024
(51) Int. Cl.: G01N 21/25, G01N 21/84, A01D 41/127, G01N 33/00, A01B 79/00, G01N 21/47, G01N 21/35, G01N 21/3563, G01N 21/3554, G01N 21/01, G01N 21/31, G01N 21/03

(54) **PORTABLE TOOL FOR ACQUISITION OF IMAGES OF SMALL SEED SAMPLE UNDER CONTROLLED IMAGING CONDITIONS**

(71) Applicant: Bayer Aktiengesellschaft, 51373 Leverkusen (DE)
(72) Inventor: Harnau, Michael, 42799 Leichlingen (DE); Roth, Franziska, 50677 Köln (DE); Steinmeister, Ingo, 51377 Leverkusen (DE); Grossmann, Katja Susanne, 68239 Mannheim (DE); Ali, Nairveen, 51105 Köln (SY); Perrody, Elsa, 31530 LASSERRE-PRADERE (FR)
(74) Representative: BIP Patents

(57) **Abstract**

The invention relates to an easy-to-handle tool for acquisition of images of small seed sample under reproducible, optimized imaging conditions, so incrementing of an image database can achieved with high data quality. The solution can be implemented as a portable device or as system comprising one or more portable imaging acquisition devices connected to a remote computer system.

## Description

The invention relates to an easy-to-handle tool for acquisition of images of seed sample under controlled reproducible imaging conditions and a control application to control of the image acquisition tool. The solution can be implemented as a portable device or as system comprising one or more portable imaging acquisition devices connected to a remote computer system.

For example, in Oilseed Rape seed hybrid production, pre-harvest treatment is used to homogenize the dryness of the crop and thousand kernel weight (TKW). It is essential that such pre-harvest treatment is done at the right moment, i.e., at ideal seed moisture content to prevent a decrease in yield and seed quality.

So far, the right time for the pre-harvest is either estimated by a field worker by assessing seed color and size visually based on experience or using laboratory equipment that cannot be used directly in the field and requires a large amount of sample material. Laboratory equipment for thermogravimetric or moisture measurement have been commonly used. However, these solutions are time-consuming, require measurements to be conducted in a laboratory (off-field), require big number of seeds per sample (more than 1000 seeds) and / or cannot not measure moisture within adequate target range (30-60 % should be covered).

In other words, available solutions do not fulfill the needs for a mobile and easy-to-use solution for field or laboratory measurement on small samples, that is comprising a limited number of seeds, for quick evaluation of seed maturity, in a non-invasive way. A solution fulfilling said needs would be of particular interest for scheduling of harvest or pre-harvest field management measures, in particular treatment and / or conducting harvest. First problem to be solved is providing an easy-to-handle, portable tool for image acquisition of seed samples under reproducible conditions for adequate computer-implemented color analysis of acquired images.

### SUMMARY OF THE SOLUTION

The solution is set out in the appended set of claims.

The term "seed" as used herein refers to the ripened ovule of gymnosperms and angiosperms, which contains an embryo surrounded by a protective cover. In particular, the term covers cereal kernels. The protective cover can comprise the seed coat (testa). Some seeds comprise a pericarp or fruit coat around the seed coat. In particular when this layer is closely adhered to the seed, as in cereal kernels, it is in some cases referred to as a caryopsis or an achene. As used herein, the term "seed coat" or "seed surface" includes a caryopsis or an achene. In practical terms, the term "seed" includes but is not restricted to anything that can be planted in agriculture to produce plants, including pelleted seeds, true seeds, plant seedlings, rootstock, plant cuttings and plant parts such as a tuber or bulb.

The seed may be of the order of Monocotyledoneae or Dicotyledoneae. Virtually the sample may be of any plant seed, such as cereals, vegetables, ornamentals, and fruits. Particular plant seeds are selected from the group of corn (sweet and field), soybean, wheat, barley, oats, rice, maize, potato, sugar cane, sugar beet, cotton, sunflower, alfalfa, sorghum, rapeseed, Brassica spp., tomato, pepper, cucumber, melon, watermelon, onion, lettuce, spinach, leek, bean, carrot, tobacco and flower seed, for example, pansy, impatiens, petunia and geranium. Crop plants can be plants which can be obtained by conventional breeding and optimization methods or by biotechnological and genetic engineering methods or combinations of these methods, including the transgenic plants and including the plant varieties which can or cannot be protected by varietal property rights.

Color of seed coat can vary with seed variety, hybrids or traits, gender (male/female), seed size (range) or shape, geographical origin area, surface roughness, ...

The solution comprises an easy-to-handle image acquisition tool (also referred to as imaging device) in combination with an application for image acquisition of a seed sample positioned in the imaging tool.

The solution is advantageous for subsequent computer-implemented image analysis of seed color and further seed parameters (averaged or range) from the image of the seed sample, in particular to determine the seed moisture, seed size and / or TKW for said sample. It is preferred that image acquisition is conducted directly in the field using a solution implemented as a portable device or system. The solution is non-invasive and requires a limited number of seeds in the seed sample.

The image acquisition tool of the solution provides reproducible optimized image acquisition conditions, so incrementing of an image database is achieved with adequate data quality for training of one or more algorithms for correlation between image features and moisture content and / or seed volume. Optimized conditions are also advantageous for use of the computer-implemented image analysis by farmer on new images acquired in a field.

### DETAILLED DESCRIPTION

Subjects of the invention and preferred embodiments are presented hereinafter.

In addition, the invention is described in detail. In this presentation and this description, no distinction is made between the individual subjects of the invention (method, system, etc..). The elucidations that follow shall instead apply analogously to all subjects of the invention, irrespective of the context in which they are made (imaging device, system, method).

The first object of the invention is a device for image acquisition of a seed sample, also referred to as imaging device, under reproducible imaging conditions, in particular lighting, eventually geometrical orientation and limited overlap of the seeds during image acquisition. The device is a portable device.

The imaging device of the invention comprises:
A seed collecting region configured to receive a seed sample;
An opaque chamber delimited by a chamber floor comprising the seed collecting region and a chamber top connected by chamber side walls between the chamber floor and the chamber top;
One or more imaging unit comprising an image sensor, preferred a camera, positioned to acquire an image of a scene comprising at least part of a seed sample positioned in the seed collecting region.

In an embodiment, the chamber side walls are dark, preferred black, to avoid reflections and / or extraneous light.

In an embodiment, the chamber can be in cylindrical, conical, pyramidal or box-shaped form. It preferably has a rectangular (especially square) cross section, and the walls extend upward from the base, with base surface and wall surface preferably running at an angle of more than 90° and less than 120° relative to one another.

In an embodiment, the chamber has a truncated pyramid form with rectangular or square base, wherein the imaging unit is placed at the truncated top of the pyramid. In an embodiment, the seed sample is made of a plurality of seeds. For imaging, the seed sample is positioned in a planar or nearly planar seed collecting region at the bottom of the chamber. In an embodiment, the surface of the seed collecting region is chosen, so the seed sample with adequate seed count nicely spreads as a monolayer, and so it can be effectively reorganized on the seed collecting region by wobbling the imaging device (means only part of the seed collecting region is covered by the seed sample). Typically seed count is chosen between 50 to 1000 seeds.

In an embodiment, the device comprises a light-tight drawer to easily introduce or remove the seed sample into/from the opaque chamber, wherein the drawer is delimited by drawer edges connected to a drawer floor building the seed collecting region.

In an embodiment, the chamber is made of lens barrel connected to a drawer cabinet, in which a drawer can be pushed in and pulled out of an enclosure of the drawer cabinet.

In an embodiment, the dimensions LxWxH (Length, Width, Height) and angle of the lens barrel are adjusted to the optimal focus settings of the image sensor. In an embodiment, the lens barrel has a truncated pyramid form with rectangular or square base. The imaging unit is positioned at the truncated top of the lens barrel; the top of the lens barrel comprises an imaging aperture in alignment with the image sensor.

In an embodiment, the lens barrel comprises an area for fixing the imaging unit and/or image sensor with precise alignment. In an embodiment, the imaging unit is a mobile phone camera. The connection of this imaging unit with the lens barrel is further described below.

In an embodiment, the top of the lens barrel comprises a pin/drilling system and a heel system for precise alignment of the device components. When assembled, the chamber is stable and twist-proof, and highly precise alignment can be achieved.

In an embodiment, the lens barrel is connected to a drawer cabinet.

In an embodiment, one or more magnet is positioned on the back of the drawer cabinet for secured closing of the drawer while it is pushed in the drawer cabinet during service or transport of the imaging device. The drawer may comprise a steel side or one or more steel plate are arranged on the drawer's side (e. g. glued-in on the drawer's back) for the magnet(s) to adhere.

In an embodiment, the magnet is positioned in a ring on the rear side of the drawer cabinet, opposite the opening area (enclosure) of the drawer cabinet, said ring holding the magnet; the ring is closed by a cap to hold the magnet in place.

In an embodiment, the drawer cabinet is stabilized using a long edge on its outside edges and opening area/enclosure.

In an embodiment, the drawer cabinet comprises an additional panel connected to the opening area (enclosure) as light trap assuring light-tightness with the drawer. The gap between the drawer and the drawer cabinet is preferably kept as small as possible, but big enough so that the drawer doesn't get stuck if dirt or the like gets in between. The drawer cabinet is configured to be easy to clean.

In an embodiment, the drawer cabinet has a cut-out in its bottom area, so one can photograph a labeled bag with the data written on it when the drawer is removed and then slide in the drawer with seeds and take a picture. An assignment of the seed images using the data noted on the bag facilitates later use of the image. In an embodiment the cut-out is delimited by a broad edge around the drawer cabinet, at the enclosure for receiving the drawer and as a light trap, on the other sides for better stability of the drawer cabinet despite the cut out.

In an embodiment, the drawer is a box-shaped container delimited by drawer edges, front, rear and side walls, connected to a drawer floor delimiting the container; the drawer is configured to slide in or out of the drawer cabinet described above, the drawer resting directly on the drawer cabinet.

In an embodiment, the drawer comprises a handle or drawer pull on the front wall for easy opening.

In an embodiment, the imaging device comprising elements made of plastic, in particular thermoplastic with good mechanical properties, that is hard and stiff. In an embodiment, plastic is chosen easy to glue for convenient assembly of the imaging device.

In an embodiment, the elements of the imaging device made of plastic are obtained by 3D-printing.

For example, Acrylonitrile Butadiene Styrene (ABS) plastic can be used; ABS is hard and stiff, easy to glue, paintable and easy to clean with water and liquid soap. Alternatively, Polylactic Acid (PLA) also widely used in 3D-printing can be used.

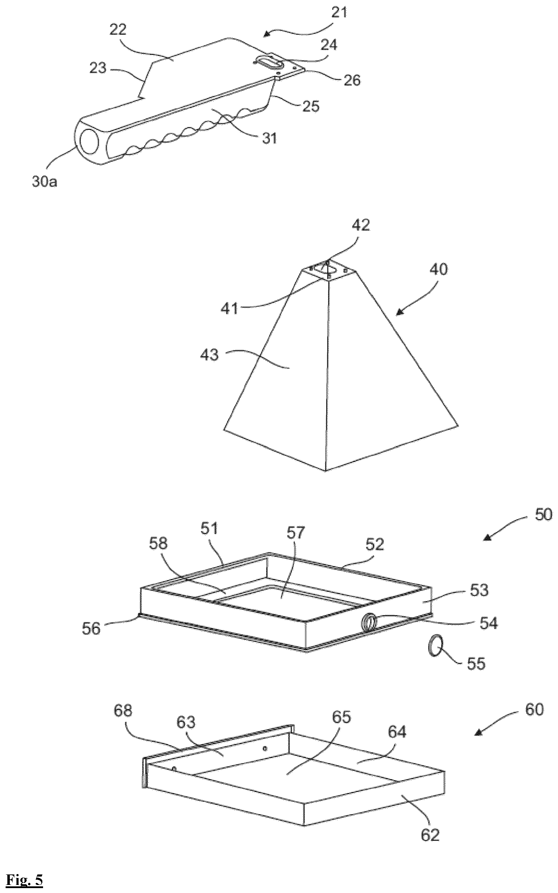

In an embodiment, the elements of the imaging devices are produced separately and assembled, for example glued. Fig. 5 shows an example of the elements of the imaging device before full assembly. Although single parts may seem wobbly, when assembled, the device was found to be very stable.

In an embodiment, wall thicknesses are chosen to be as thin as possible to ensure sufficient stability of the assembled device. In an embodiment, wall thickness is chosen from 0.5 - 3 mm. In an example, wall thickness for the lens barrel was chosen 1.5 mm. The resulting low weight for the device benefits the user when handling the device to avoid premature wrist fatigue. In the example described below, the complete device was constructed with a total weight of 341g without smartphone, and 497g inclusive smartphone type iPhone SE2020.

In an embodiment, the drawer comprises one or more magnetic holders/plates on the rear wall, so it can open easily but does not fall out accidentally out of the chamber, for example when the device is being transported. In an embodiment, the drawer comprises a recess for a steel plate (magnetic system) in the rear area and a steel plate positioned in the recess. In an example, a steel plate is introduced, optionally glued, in the recess. Alternatively, or additionally the steel plate can hold in position by an insert inserted in the drawer to receive the seed sample. An insert is further described below.

In an embodiment, the drawer comprises an aperture for pushing out the insert if needed. With a piece of round material through the aperture, user can push out the insert from below, if necessary, without fiddling around and breaking out the thin side walls of the drawer. The aperture also serves as a drain when cleaning the drawer.

Since the insert and / or the drawer become dirty over time, user can completely remove the drawer including the insert from the drawer cabinet. Cleaning can be achieved with water and liquid soap.

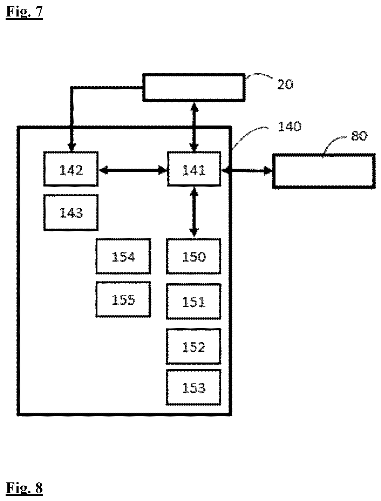

In an embodiment, the front wall of the drawer comprises a panel overlapping at least the upper part of the front wall of the drawer and adjusted to overlap the enclosure of the drawer cabinet for light trap (Fig. 7).

In an embodiment, the drawer is designed in such a way that it not only rests on a table when it is pushed in the device, but also in the laboratory when it is removed from the device. The height, width and depth of the drawer are adapted to the drawer cabinet and the function of the insert.

For imaging, a seed sample is placed on a seed collecting region. The seed collecting region may be the bottom of the chamber, the bottom of a drawer to be positioned in the chamber or the bottom of an insert to be positioned in a drawer for more convenient handling.

In an embodiment, the seed collecting region has a round, oval, elliptical, angular (triangular, tetragonal, pentagonal, hexagonal or generally n-angular, with n being an integer greater than or equal to 3) cross section framed by walls. In an example, the cross section is rectangular, for example square.

In an embodiment, the seed collecting region is planar/flat, rough, or structured. Structured seed collecting region can be advantageous for better distribution of the seeds over the whole seed imaging area in the drawer and /or for maintaining constant seeds position and orientation during image acquisition.

In an embodiment, the drawer is configured to build a volume in which the seeds spontaneously nicely distribute in the seed collecting region, when the seed sample is poured into or moved in the drawer or insert. For this purpose, the framing walls delimiting the seed collecting region are at least partly tilted towards said seed collecting region.

In an embodiment, the tilted framing walls are planar or structured. For example, the tilted framing walls can be structured with concentric rills centered on the collecting region.

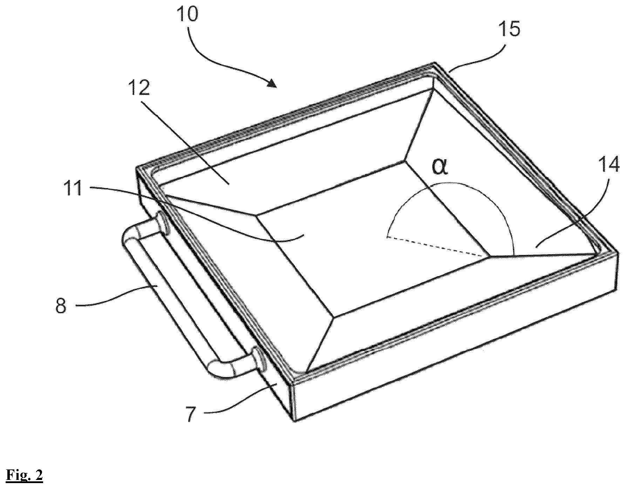

In an embodiment, tilted framing walls are at least partly tilted with a tilting angle α to the planar the seed collecting region from 140 to 170 degrees, preferably 150 to 170 degrees (Fig. 2). In an example for OSR seeds, a tilting angle of 165 degrees was used.

In an embodiment, the drawer is configured for smooth complete pouring of (small) seeds out of the drawer, for example back into a seed bag without losing them.

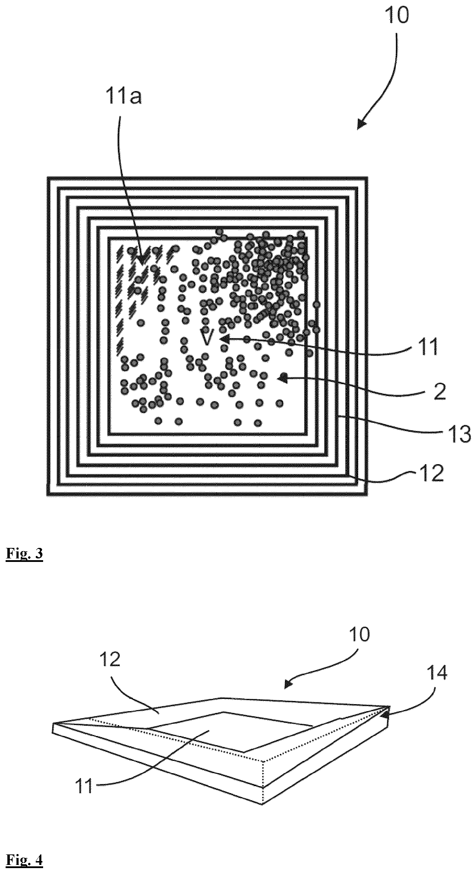

In an embodiment, two of the tilted walls are configured to build a pouring corner, so the pouring corner smoothly guides the seeds up the drawer rim. In other words, the drawer and / or an insert for the drawer is asymmetric, the tilted walls of the pouring corners having dimensions and angles, so the pouring corners reaches the drawer upper edge (Fig. 2, Fig. 4).

In an embodiment, the plane collecting region is provided by an insert adapted to the drawer, said insert comprising framing walls tilted towards the planar seed collecting region with tilting angle α. In an embodiment, the insert is sunk so far into the drawer that when filling in the seeds and moving the drawer back and forth for better and more even distribution, no seed flies out of the drawer and / or fall back to the seed collecting region. In an embodiment, at least part of the rim of the insert walls is below the drawer rim.

In an embodiment, the insert is designed in such a way that it can be removed, replaced, renewed, or exchanged at any time.

The skilled person will appreciate that the dimensions and angles of the device can be adapted so that seed/product rolls loosely on the surface of the seed collecting region again and again upon wobbling the device. Generically, the solution is usable for separable seeds, so good quality color images of a seed sample positioned on a background (seed collecting region of an imaging device) can be acquired. "Separable seeds" as used herein relates to the ability for a seed sample comprising a plurality of seeds to build a monolayer on a seed collecting region. "Separable" is opposed to aggregated non-separable seeds in very humid state.

In an embodiment, the seed collecting region is blue. Blue color was found to be advantageous for easier image segmentation of seeds from background. In an embodiment, the drawer or the insert is blue. Blue color can be obtained either by blue painting of the surface or by using blue material to make the insert.

In an embodiment, the seed collecting region has a glossy, matte, rough or structured surface. The collecting region preferably has a rough or structured surface in order to avoid direct reflections of lighting from the collecting region onto the image sensor.

In an embodiment the insert is made of rigid or semi-rigid synthetic material for easy cleaning.

The imaging device comprises an imaging unit.

In an embodiment, the imaging unit further comprises one or more lighting source(s) directed to the collecting region, such that light (electromagnetic radiation in the infrared, visible and/or ultraviolet region of the spectrum) is scattered/reflected by the illuminated collecting region in the direction of the imaging unit.

In an embodiment, such lighting source(s) can be activated by way of an activation lamp function of the imaging unit and / or image acquisition with a camera flash function.

In an embodiment, lighting source(s) is preferably mounted laterally alongside the imaging unit, such that no shadows of the imaging unit are thrown onto the collecting region.

It is also conceivable to position an illumination source beneath the collecting region and/or alongside the collecting region, which illuminates the collecting region "from the side", whereas an imaging unit produces one or more digital images "from the top".

It is conceivable that multiple illumination sources illuminate the collecting region from different directions.

It is conceivable that the electromagnetic spectrum of the light emitted by the lighting sources for illumination is matched to the color of specific seeds and/or the color of the collecting region, in order to produce a maximum contrast between the seeds and the background against which they are imaged.

In a preferred embodiment, the illumination and/or color and/or surface characteristics of the collecting region is chosen such that the electromagnetic radiation used for illumination is thrown back (reflected or scattered) more significantly by the sample than by the collecting region. In an alternative embodiment, the illumination and/or color and/or surface characteristics of the collecting region is chosen such that the electromagnetic radiation used for illumination is thrown back (reflected or scattered) more significantly by the collecting region than by the sample. Both cases ensure high contrast. A high contrast facilitates the computer-implemented identification of (specific) sample from the surface of the collecting region (also referred to as background in image analysis).

The term "light" and "illumination", incidentally, is not supposed to mean that the spectral region is limited to visible light (about 380 nm to about 780 nm). It is likewise conceivable that illumination is conducted using electromagnetic radiation having a wavelength below 380 nm (ultraviolet light: 100 nm to 380 nm) or above 780 nm (infrared light: 780 nm to 1000 µm). The image sensor and the optical elements are matched to the electromagnetic radiation used.

The device of the invention further comprises one or more imaging units. Such imaging unit can be used to generate/capture digital images. An imaging unit comprises an image sensor and optical elements.

In an embodiment, the image sensor is a device for recording two-dimensional images from light by electrical means. This typically comprises semiconductor-based image sensors, for example CCD (CCD = *charge-coupled device*) or CMOS sensors (CMOS = *complementary metal-oxide-semiconductor*)*.* The optical elements (lenses, stops and the like) serve for maximum sharpness of imaging of the object of which a digital image is to be generated on the image sensor.

In an embodiment, the imaging unit is positioned such that the entire or at least part of the seed collecting region is imaged on the image sensor. It is conceivable to use multiple imaging units that image different regions of the collecting region on the respective image sensor. In the case of such a use of multiple imaging units, it is advantageous when the regions imaged at least partly overlap, to be able to generate an overall image in a simpler manner from the individual images at a later juncture.

In an embodiment, the imaging unit is set with fixed exposure time and focus for acquisition of multiple images. Use of fixed settings were found to improve reliable model-based image analysis.

In an embodiment, camera focus is centered.

In an embodiment, a diffuse lens may be used in the slotted aperture for the camera and flashlight.

In an embodiment, the imaging unit is positioned at the chamber top.

In an embodiment, the imaging unit is detachable from the chamber top.

In an embodiment, the device comprises an element for positioning and fixing the detachable imaging unit or part thereof. The person skilled in the art will appreciate that the whole or part(s) of the imaging unit can be detachable. For example, light source(s) can be non-detachable.

In an embodiment, the chamber top is configured to accommodate the detachable imaging unit in a fixed position relative to the seed collecting region.

In an embodiment, the device comprises a holder for positioning and (reversibly) fixing the detachable imaging unit or part thereof. The holder is preferably at a defined and constant distance from the collecting region, and hence ensures a defined and constant distance between the image sensor and the collecting region. This facilitates the focusing of the collecting region. The holder also allows precise alignment of the imaging unit/sensor with the seed collecting region for best quality imaging.

In an embodiment, the element for positioning and fixing the detachable camera is formed to allow quick and easy placing and removal of the imaging unit from the chamber.

In an example, flexible holder is used for easy insert and removal of different imaging units, similar a smartphone shield casing used for smartphone protection.

In an embodiment, the imaging unit can be the camera and lighting of a smartphone or mobile phone.

In an embodiment, the imaging unit is positioned at the chamber top. In other words, the image sensor, such as a camera, is positioned to acquire an image of the collecting region from above.

The holder holds the imaging unit in position at the top of the chamber/lens barrel underneath.

In an embodiment, the size of the upper surface of the holder is adapted to receive the imaging unit. In case imaging unit is a mobile phone, the holder comprises a mobile phone case fixed (e. g. glued) to the upper surface of the holder. In an embodiment, the upper surface comprises a slope, designed so that the mobile phone case can be bent away when the mobile phone is removed. Commercially available flexible or semi-flexible phone cases commonly made of hard plastic or silicone matching used mobile phone were used protection and connection of the mobile phone to the holder.

In an embodiment, the holder comprises an aperture for the mobile phone camera and flash. In an embodiment, the hold is adjusted with a slope that corresponds to the opening angle of the mobile phone lens and geometry of the chamber/lens barrel. The slope stabilizes the chamber/lens barrel wall when the device is assembled. In an embodiment, a pin/drilling system is used for optimal bonding and perfect alignment of the individual components with one another.

To avoid undesired incidence of light between the mobile phone, the mobile phone case and the holder, a shoulder can be constructed around the aperture for the camera and flash (slotted aperture). The slotted aperture can be used to center the cell phone case when fixing it to the upper surface of the holder.

In an embodiment, the holder comprises a flattened side to support the ball of the hand, for better control and handling of the device. A further object of the present application is a system comprising at least one imaging device as described above and a control unit comprising one or more processors configured for conducting the image acquisition by the image acquisition device.

In an embodiment, the system further comprises peripherals.

In an embodiment, the one or more processors are configured for:
Prompting user on the peripheral for introducing or confirming an identificator for the seed sample;
Prompting user on the peripheral that (new) sample can be introduced;
Triggering the imaging unit of the imaging device to capture images of the seed collecting region;
Checking positioning of the seeds on the seed imaging surface by way of activating a light source and / or a function of test imaging; and / or
Prompting user on the peripheral that image acquisition is completed.

The imaging device of the invention further comprises a control unit comprising one or more processors configured to trigger the at least one imaging unit to record digital images. Images may be generated regularly or irregularly. In an embodiment, the one or more processors can be configured to capture single or a plurality of images,

For better results, it is preferred that for each sample images from all sides of the seeds are acquired. For this purpose, the user shall wobble the image acquisition tool between two image acquisition. In an embodiment, the one or more processors are configured to prompt the user for wobbling between two image acquisitions, preferably until a predefined number of images are acquired for the sample.

It is preferred that 2 to 10 images are acquired; most preferred 5 images per sample are acquired.

In an embodiment, the one or more processors can be configured to route the one or more images to a transmitter unit. Alternatively, the one or more processors can be configured to average image data of the multiple images for the sample and averaged image data is routed to the transmitter unit.

In an embodiment, the control unit is configured to control the lighting sources, e. g. for image acquisition, test imaging function and / or checking adequacy of imaging conditions, etc...

In case a mobile phone comprising several cameras is used, the control unit is configured to choose the adequate camera based on fixed settings.

The control unit can be configured to route the images recorded and / or information relating to the one or more images to a transmitter unit to send them via a radio network to one or more remote computer system comprising an image data repository and / or one a more processors configured for image storage and analysis.

This information may be the one or more images themselves. The images can be analyzed (in an automated manner) by the computer system(s).

The transmitter unit may be configured such that it transmits information via a mobile communications network (e.g. GSM: Global System for Mobile Communications, GPRS: General Packet Radio Service; UMTS: Universal Mobile Telecommunications System, LTE: Long Term Evolution), via a WLAN (Wireless Local Area Network), via Bluetooth, via DECT (Digital Enhanced Cordless Telecommunications) via a low-power wide-area network (LPWAN or LPN)), for example a Narrow Band IoT network and/or via a combination of different transmission routes.

In an embodiment, the transmitter unit is configured such that it transmits information via a short-range (radio) link to a base station, from which the information is then transferred to cable and/or passed on via a long-range radio connection. The term "short-range" means that the distance between transmitter and receiver is preferably not greater than 5 km, more preferably not greater than 1 km. A greater distance than the specified maximum distance would mean that faultless transmission of data from the transmitter to the receiver is no longer assured. The term "long-range" means that the distance between transmitter and receiver is preferably greater than 1 km, more preferably greater than 5 km. Communication via a short-range link can be achieved, for example, by means of radio, ultrasound and/or (laser) light.

Each acquired image is preferably assigned to a geographical location at which seed sample was collected. In case image is acquired in the field, this is typically the position where the imaging device generates images. It may alternatively be a position in the environment of a device (for example the position of a base station with which the respective device is connected via a radio connection), or the position may be fuzzy in that, for example, a region on the Earth's surface where the device is present is specified (in the form of a circle with a defined radius).

In a preferred embodiment, the system of the invention comprising means of determining the geographical position of the imaging device at image acquisition.

It is conceivable that the imaging device comprises a GPS sensor (GPS: global positioning system) or another sensor of a global satellite navigation system (GNSS) with which the position of the device can be ascertained. One advantage of determining position by means of a global satellite navigation system is high accuracy. Disadvantages are the additional component costs and the comparatively high energy demand.

It is also conceivable that the position is determined by means of the radio cell to which the transmitter unit is connected. Such a solution typically has a lower accuracy than determination of position but means lower component costs and a lower energy demand.

In mobile communications, the simplest way of determining position is based on the fact that the cell in which a transmitter unit is present is known. Since, for example, a switched-on mobile phone is connected to a base station, the position of the mobile phone can be assigned to at least one mobile communications cell (cell ID). It is also possible to proceed analogously with a device of the invention.

With the aid of GSM (Global System for Mobile Communications), the position of a transmitter unit can be determined accurately to several hundred meters. In towns, the position can be determined accurately to 100 to 500 m; in rural areas (in which the density of base stations is lower), the radius is increased to 10 km or more. If the information is combined with the TA parameter (TA: Timing Advance) via the cell ID, the accuracy can be increased. The higher this value, the greater the distance of the transmitter unit from the base station. It is possible to locate a transmitter unit even more accurately by the EOTD method (EOTD: Enhanced Observed Time Difference). This determines the differences in transit time of the signals between the transmitter unit and multiple receiver units.

In one embodiment, information is ascertained, and position is determined by means of the Sigfox grid. Sigfox is a low-power wide-area network (LPWAN) and is specifically designed for small data packets and very power-saving operation. Sigfox can communicate over long distances without disruption. The range of a single base station, which can control up to one million transmitter units, is 3 to 5 km in areas of high population density and 30 to 70 km in rural areas. In the case of Sigfox, the data packets are received by all base stations in the transmitter region. This can be used to determine the position of a transmitter unit.

It is also conceivable that the position of an imaging device is detected when it is registered. In such a case, one step of the registration is the association of the imaging device and position. It is conceivable that a user, by means of a mobile computer system (e.g. a smartphone or a tablet computer or the like), detects an unambiguous identifier of the device and associates it with position information. The unambiguous identifier serves for identification of the device when it is registered. The unambiguous identifier may be a number or an alphanumeric code or a binary code or the like which is mounted on the device or recorded in a data storage means of the device. The unambiguous identifier can be detected, for example, by input via an input device (e.g. a keyboard, a touch screen, a microphone (by speech input) or the like) into the mobile computer system. Preferably, the unambiguous identifier takes the form of an optically readable code (for example a barcode or matrix code or the like) or of an electronic storage means that can be read out by radio (e.g. in the form of an RFID tag) or the like. This has the advantage that the unambiguous identifier can be read out automatically with the mobile computer system, and input errors (as in the case of a user typing it in using a keyboard) are avoided. An optical code can be detected, for example, with a camera that may be part of the mobile computer system. In a further step, the position is determined. It is conceivable that the means of determining position are provided by the user's mobile computer system. The mobile computer system may, for example, be a smartphone with which the position is determined via the radio cell to which the smartphone is connected, or with a GPS sensor associated with the smartphone.

It is preferred that metadata associated with acquired image of a seed sample comprises geographical location at which the sample was collected. The person skilled in the art will appreciate that such location can be collected automatically by the imaging device or introduced by user.

If the unambiguous identifier has been detected and the position determined, these pieces of information are associated with one another. The association assigns the device to a position. It is conceivable that the associated information is transmitted via a network to an external computer system and stored therein. It is also conceivable that the associated information is stored on the user's mobile computer system.

On registration, preference is given to additionally associating the unambiguous identifier of the device with an unambiguous identifier of the user, such that the user is assigned an individual device (or multiple devices) with a defined position. As a result of this association, the user is preferably able to receive only images from the device assigned to the user.

The image data transmitted by the imaging devices by means of the corresponding transmitter units to a computer system can be processed, analyzed, archived and/or issued to a user by way of one or more peripherals.

Further information related to an acquired image may comprise geographical location, day and time of image acquisition; said information and acquired images are together referred to as image data. Further data related to the image may be introduced by the user into the imaging device and / or the computer system by way of peripheral, and / or collected from a data repository based on geographical location. Examples for such further data are field name, genotype of the seeds, weather condition at the location, time and day of image acquisition, etc...

In an embodiment, the imaging device of the invention further comprises a handle to hold the device. Most preferred the handle is ergonomically shaped for secure handling of the device. The handle may comprise an eyelet for a shoulder or wrist strap. The handle can left-handed or right-handed.

In an embodiment, the handle is part of the holder.

In an embodiment, the handle is hollow to save weight. The hollow space in the handle can be used to hold a power bank (to supply power to the mobile phone) and / or to hold cables.

In an embodiment, the handle is arranged offset to the mobile phone (not in the middle), so user can access the entire surface of the mobile phone without much effort in a balanced way for ergonomic use. Edges have been broken and rounded off for security and ergonomics.

In an embodiment, the imaging device can comprise sensors for controlling positioning of the seed collecting region, preferably perpendicular or nearly perpendicular to the vertical direction of gravity, to avoid seed movement during image acquisition. Additionally and / or alternatively handle is configured to support horizontal positioning of the seed collecting region during acquisition. The horizontal runs perpendicular (at an angle of 90°) to the direction of gravity. The seed collecting region is thus arranged such that it and the horizontal form an angle greater than zero and not more than 90°. The seed collecting region is preferably arranged such that it and the horizontal form an angle in the range from 2° to 88°, preferably in the range from 5° to 80°, even more preferably in the range from 10° to 70°. Seed sample can thus run off the surface in a problem-free manner.

In an embodiment, image acquisition comprises placing a seed sample on the seed collecting region of the imaging device, closing the opaque chamber, typically by way of closing the drawer, and triggering the imaging unit to capture one or more images of a scene comprising at least whole or part of the seeds collecting region, preferably at least the whole seeds collecting region.

In an embodiment, the scene comprises the seed collecting region and at least part of the tilted walls delimiting the seed collecting region.

In an embodiment, image acquisition can further comprise one or more of:
Prompting user for introducing or confirming metadata related to the sample, such as but not limited to identificator for seed sample, field, day and time, etc;
Providing an image preview of the scene to be captured to the user by way of causing activating lighting sources for display of the illuminated scene on a display screen,
Providing a test image by way of an imaging test imaging function activating the lighting source and the imaging unit in a camera flash function.
Causing checking adequacy of imaging conditions by running a function of comparing imaging conditions measured by the imaging device with predefined thresholds;
Prompting the user to confirm correct imaging conditions or image acquisition;
Prompting user that image capture/acquisition is completed; and / or
Prompting user for introducing new sample at same or different location;
Causing transmission of acquired image data, typically image together with related metadata, to one or more processors configured for image storage, image treatment and / or image analysis.

In an embodiment, metadata can be entered manually by the user. In an embodiment, metadata can be introduced by way of photographing sample bag, automatic reading of information, e. g. using a scanner, and introducing in corresponding fields.... Most relevant metadata are one or more of country, field name, genotype, modality, measurement day/time, batch ID, plot row and number, weather data, replicate number... In an embodiment, QR codes or labels for samples storing metadata for the sample can be used. In an embodiment, every plot in a field has a QR code containing all necessary metadata generated with a software application. Alternatively, sample bags may have labels storing metadata.

Correct imaging conditions herein may refer to adequate seed sample size, adequate positioning of the seeds in the seed collecting region, adequate orientation of the imaging device based on sensor information provided by the imaging device, adequate lighting conditions, clean, dirty or damaged optics for imaging unit and the like.

In an embodiment, the control unit is configured for causing display of the image preview or of the test image to the user for quality check, prompting the user for instruction of capturing one or more images, storing and / or of transmitting acquired image data via a network to one of more computer systems comprising one or more processors configured for storage of image data in an image depository and/or treatment of the transmitted image data.

In an embodiment, image acquisition further comprises automatically triggering the transmitter unit to send the referred to as image data via a network to the one or more computer systems.

In embodiment, one or more of the sub-processes related to image acquisition are implemented in a control application.

The imaging device and / or the computer system can comprise peripherals for displaying test images, acquired images and / or results of image analysis described above.

In an embodiment, the device and / or the computer system can comprise an image data repository for storage of the seed sample images. Said image repository can be part of the device and / or part of the remote computer system.

A "computer system" is a system for electronic data processing that processes data by means of programmable computation rules. Such a system typically comprises a "computer", that is a unit which comprises one or more processors for performing logic operations, and peripherals.

In computer technology, "peripherals" refer to all devices which are connected to the computer and serve for control of the computer and/or as input and output devices. Examples thereof are monitors (screen), printers, scanners, mice, keyboards, drives, cameras, microphones, loudspeakers, etc. Internal ports and expansion cards are also considered to be peripherals in computer technology.

Today's computer systems are frequently divided into desktop PCs, portable PCs, laptops, notebooks, netbooks and tablet PCs and so-called handhelds (e.g. smartphones); all these systems can be utilized for execution of the invention. In other words, processors configured to run the methods described in the present application can be integrated into the (portable) imaging device and / or parts of a remote computer system.

Inputs into the computer system are made via input devices, for example a keyboard, a mouse, a microphone and/or the like. "Input" shall also be understood to mean the selection of an entry from a virtual menu or a virtual list or clicking on a selection box and the like.

A system can comprise several devices that are assigned to a computer system. The devices and the computer system can be connected to one another via a network, such that the devices can transmit information (data, images, status information, sensor data or the like) to the computer system. It is also conceivable that the system is configured such that the computer system can transmit information or control commands to the devices. The network via which the computer system and the devices are connected to one another can be at least partly a radio network. Information can be transmitted from a device (an imaging unit or a repository comprising images data acquired using an imaging unit) via a transmitter unit by radio to a base station, from which it is passed onward (optionally via further stations) by radio and/or via cable to the computer system comprising one or more processors configured for the treatment and/or analysis of the transmitted information.

A computer system can comprise a data repository, data library or data archive, that is a data set isolated to be mined for data reporting and analysis. The data repository can be a large database infrastructure - several databases - that collect, manage, and store data sets for data analysis, sharing and reporting.

The imaging device of the invention allows convenient image acquisition on the field or more generically in an area or a sub-area under optimized standard conditions.

An "area" is understood to mean a spatially delimitable region of the Earth's surface on which the plants of interest grow. Preferably, the area is at least partly utilized agriculturally in that crop plants are planted in one or more fields, are supplied with nutrients and are harvested. The area may also be or comprise a silviculturally utilized region of the Earth's surface (for example a forest). Also gardens, parks or the like in which vegetation exists solely for human pleasure are covered by the term "area".

An area preferably includes a multitude of fields for crop plants. In a preferred embodiment, an area corresponds to a growing area for a crop plant (for definition of a growing area see, for example, Journal für Kulturpflanzen, 61 (7). p. 247-253, 2009, ISSN 0027-7479). In another preferred embodiment, an area corresponds to a biome (for definition of equivalent German term Boden-Klima-Raum see, for example, Nachrichtenbl. Deut. Pflanzenschutzd., 59(7), p. 155-161, 2007, ISSN 0027-7479).

A "sub-area" is understood to mean a preferably contiguous region within an area. A sub-area is preferably one or more fields in which a specific crop plant is being grown. The sub-area is preferably being farmed by a farmer having registered access to a portable imaging tool and optionally one or more plant analysis devices.

Seed moisture and / thousand kernel weight of a seed sample can be ascertained for an area or for a sub-area. Determination for a sub-area is preferred when variability of the indirectly measured seed moisture and / thousand kernel weight ascertained for the whole area exceeds a predefined threshold and or measurement ascertained for another sub-area of the same area does not exceed the damage threshold.

The ascertaining of seed moisture and / or thousand kernel weight range is useful from an agricultural management point of view. A seed moisture and / or thousand kernel weight range indicates the seed maturity and can be used for scheduling of an area management task such as pre-harvest treatment and / or harvest, over and above which aera management task is economically viable. Outside range, loss of yield and / or decreased seed storability and viability is to be expected.

For assessment of the seed moisture and / or thousand kernel weight in an area, at least one imaging device is used.

For assessment of the seed moisture and / or thousand kernel weight in an area, at least one seed sample and at least one image of said seed sample is needed.

In an embodiment, more than one images are acquired for each seed sample, eventually a set of images is acquired. In an embodiment, image analysis is conducted on each image of the set; results of image analysis can be averaged for the seed sample.

In an embodiment, more than one seed sample are imaged for evaluation of seed maturity in an area. In an embodiment, ascertaining of seed moisture and / or thousand kernel weight for the area can be obtained by way of averaging the results for image analysis for all the seed samples of the area and / or determining a range of seed moisture and / or thousand kernel weight for the area. In an embodiment, a distribution of seed moisture and / or thousand kernel weight for the area can be determined.

In an embodiment, the system can be configured to define one more sub-areas and determine an average or a range of seed moisture and / or thousand kernel weight for the sub-area.

Defining sub-areas is of particular interest, in case the determined distribution of seed moisture and / or thousand kernel weight for the area is above a predefined threshold or ascertained range seed moisture and / or thousand kernel weight for the area is too broad. Defining sub-areas can be used for area mapping, wherein results for one or more subareas are shown.

In an embodiment, the system is configured to compute discrete locations or a trajectory comprising discrete locations (also called an acquisition path) for image acquisition by the user within the area and / or sub-area, for example in consideration of topography, shadowing, ponding information of the area/sub-area at stake. In this case, the system can be configured to transfer the computed acquisition path and/or discrete locations to the control unit. Control unit can be configured for causing display of the acquisition path and/or causing prompting the user for collecting and introducing field sample according to acquisition path.

In an embodiment, the control unit comprises one or more processors configured for conducting the image acquisition. In an embodiment, the one or more processors of the control unit can be configured for one or more of the following purposes:
Prompting user for introducing or confirming identificator for seed sample, field, day and time, etc;
Controlling lighting sources and / or imaging unit for preview or test function described above;
Prompting user that image acquisition is completed;
Prompting user for introducing new sample in the collection region at/from the same or another location optionally according to a computed acquisition path.

In an embodiment, the one or more processors can be configured to control adequacy of imaging conditions and cause an alarm signal triggered by inadequate condition for image acquisition, for example insufficient contrast, inadequate sample, default in lighting, sharpness, etc. Control of adequate conditions may be conducted on an image preview, a test image and / or the acquired image.

The information transmitted by one or more imaging devices described above to a computer system, by means of the corresponding transmitter units, can be processed, analyzed, archived and/or issued to a user therein.

Further object of the application is a method comprising:
Placing a seed sample on the seed collecting region of an imaging device as described above;
Triggering the imaging unit to generate/capture one or more images of a scene comprising at least part of the seeds collecting region, preferably at least the whole seeds collecting region;
Acquiring the one or more images.

In an embodiment, the method further comprises one or more of:
Prompting user for introducing seed sample and optionally metadata related to the seed sample;
Causing the imaging device generating an image preview and / or a test image of the seed collecting region by way of activating the lighting source and / or a function of test imaging;
Causing checking adequacy of imaging conditions by displaying image preview and / or test image to the user, alternatively by running a function of comparing imaging conditions with predefined thresholds; and / or
Prompting user that image acquisition is completed;
Causing transmission of acquired image data to one or more processors configured for image storage, image treatment and / or image analysis.

The invention is elucidated in detail hereinafter by figures and examples, without any intention to restrict the invention to the features and combinations of features specified in the figures and examples.

The figures show:
Figure 1A and 1B show 3-dimensional schematic views of image acquisition device 1 (also referred to as imaging device) from above front with closed or open drawer.
Figure 2 shows a 3-dimensional view from above of an empty drawer and insert.
Figure 3 show a schematic view from above of an insert containing a seed sample.
Figure 4 shows a 3-dimensional view from the side above of an asymmetric insert.
Figure 5 shows a 3-dimensional schematic view from the side of the component of the acquisition device before assembling.
Figure 6 shows a 3-dimensional schematic view of the drawer from above front side.
Figure 7shows a detail of the light trap when the device 1 is assembled and the drawer 60 is closed in the drawer cabinet 50.
Figure 8 shows a diagram of the system comprising the image acquisition device.
Figure 9 shows a flow diagram of a method for use of the image acquisition device for imaging seeds.

### References:

Image acquisition device (1)
Seed / Seed sample (2)
Chamber wall (5) delimiting a chamber room (6)
Drawer (7)
Drawer handle (8)
Insert (10)
Seed collecting region (11)
Structured surface of seed collecting region (11a)
Tilted walls (12)
Rills of tilted walls (13)
Pouring corner (14)
Drawer edge/rim (15)
Imaging unit (20, not shown)
Holder for imaging unit (21)
Upper surface of the holder (22)
Mobile phone case (not shown)
Slope for easy removal of mobile phone (23)
Slotted aperture for mobile phone camera and flash (24)
Slope supporting lens barrel (25)
Pin/drilling system and a heel system for fixation of lens barrel (26)
Handle (30) / Hollow handle (30a)
Flattened side (31)
Lens barrel (40)
Pin/drilling system and a heel system for fixation to holder (41)
Imaging aperture (42)
Lens barrel wall (43)
Drawer cabinet (50)
Enclosure (51)
Side wall of the drawer cabinet (52)
Back wall of the drawer cabinet (53)
Magnet ring (54)
Cap for magnet ring (55)
Stabilizing edge (56)
Cut-out (57)
Edge of the cut-out (58)
Light trap (59)
Drawer (60)
Drawer handle (61)
Drawer back (wall) (62)
Drawer front (63)
Drawer side walls (64)
Drawer floor/bottom (65)
Drawer aperture (66)
Steel plate and recess (67)
Drawer light trap panel (68)

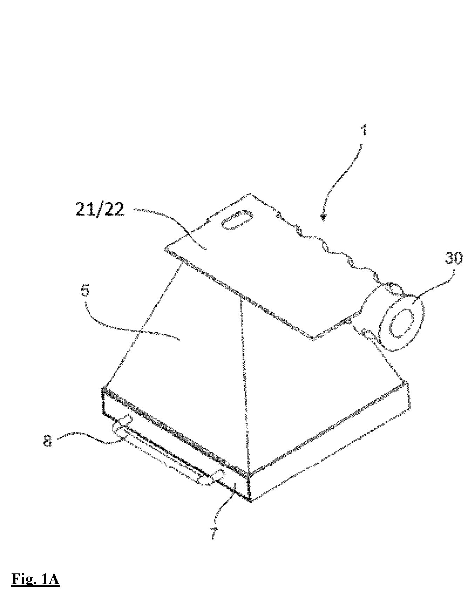

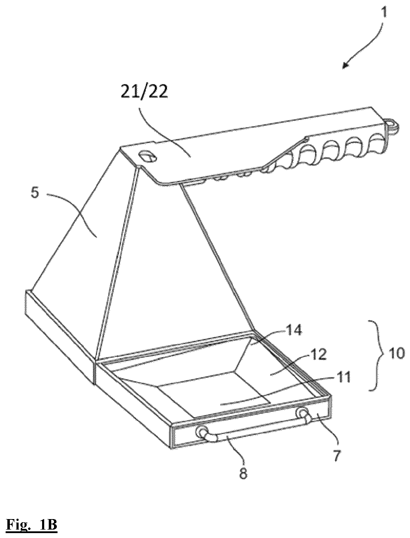

Figure 1A and 1B show 3-dimensional schematic views of device 1 comprising a drawer 7. The chamber room 6 delimited by chamber walls 5 has a truncated pyramid form with square base. In Fig. 1A drawer 7 is inserted in a drawer cabinet covering the floor of chamber room 6 (not shown), drawer front building a light trap with drawer cabinet (further detailed in Fig. 7); drawer 7 can be opened/closed in a drawer cabinet by way of drawer handle 8. At the top of chamber room 6 (truncated top of the pyramid), an imaging unit 21 (not shown) can be positioned in a detachable way using holder 21. Imaging unit 20 for use in the arrangement of Fig. 1 is a smartphone (not shown). For convenient handling of the device, the holder 21 is connected to an ergonomic handle 30. An eyelet 31 (not shown) can be added to handle 30 to insert a strap for easy carrying of the device 1 as a portable device 1. Fig. 1B shows the imaging device 1 with open drawer 7, wherein insert 10 is positioned in drawer 7, ready to receive a seed sample in seed collecting region 11 delimited by tilted walls 12. Pouring corner 14 is positioned at the back of the drawer 7 for easy removal of the seed sample later when drawer 7 is taken out of the drawer cabinet.

Figure 2 shows details of drawer 7 wherein insert 10 is positioned, said insert 10 comprising a planar seed collecting region 11 delimited by framing walls 12 tilted with tilting angle α to the seed collecting region 11. Drawer 7 can be opened/closed by way of drawer handle 8. The drawer 7 and insert 10 of Fig. 2 are square shaped. Insert 10 is sunk so far into the drawer 7 that at least part of the rim of the insert walls 12 is below drawer rim 15, except for the pouring corner 14.

Figure 3 shows a schematic view from above of a symmetric insert 10 containing a seed sample, wherein seeds 2 are placed in the seed collecting region 11 showing structured surface 11a. Seeds are spread over the collecting region 11. The insert shows tilted walls 12 comprising rills 13.

Figure 4 show a detailed 3D schematic view sideways from above of an asymmetric insert 10 with pouring corner 14, seed collecting region 11 framed by tilted walls 12. Insert was produced by 3D printing.

Figure 5 shows a 3-dimensional schematic view from the side of the components of the acquisition device before assembling. Mobile phone (imaging unit) and mobile phone case are not shown. Holder 21 for imaging unit comprises an upper surface 22 for fixing mobile phone case (not shown). The upper surface 22 comprises slope 23 designed so that the mobile phone case can be bent away for easy removal of mobile phone (not shown) and a slotted aperture 24 for mobile phone camera and flash, wherein a shoulder is constructed around the aperture 24. The slotted aperture 24 can be used to center the cell phone case when fixing it to the upper surface of the holder 22. The holder 21 further comprise is a slope 25 that corresponds to the opening angle of the mobile phone lens and geometry of the chamber/lens barrel 40. The slope 25 stabilizes the chamber/lens barrel wall 43 when the device is assembled. A pin/drilling system 26 is used for optimal bonding and perfect alignment of the individual components with one another. Handle 30a is hollow to save weight. The hollow space in handle 30 can be used to hold a power bank (not shown) and / or to hold cables (not shown). A flattened side 31 is used to support the ball of the hand, for better control and handling of the device during service. Lens barrel 40 comprises lens barrel walls 43; the dimensions LxWxH and angle of the lens barrel walls 43 are chosen in consideration of the optimal focus settings of the image sensor of the mobile phone; they form a truncated pyramid with square base. The truncated top of the pyramid comprises elements of pin/drilling system and a heel system 41 for fixation to the corresponding elements of pin/drilling system and a heel system 26. Drawer cabinet 50 comprises back wall 53, side walls 52, a floor and an enclosure 51 to engage drawer 60. Back wall 53 comprises a magnet ring 54, delimiting a cavity wherein a magnet is engaged at assembly maintained in the cavity by closing with a cap 55. For better stability the lower edge of the drawer cabinet is strengthened to form stabilizing edge 56. Stabilizing edges 56 also allows laying drawer 60 flat on a table. In example shown, the floor of drawer cabinet 50 comprises a cut-out 57 delimited by edges of the cut-out 58. The enclosure 51 is configured to fit with the light trap panel 68 of the drawer front 63 when closed (not shown) to from a light trap.

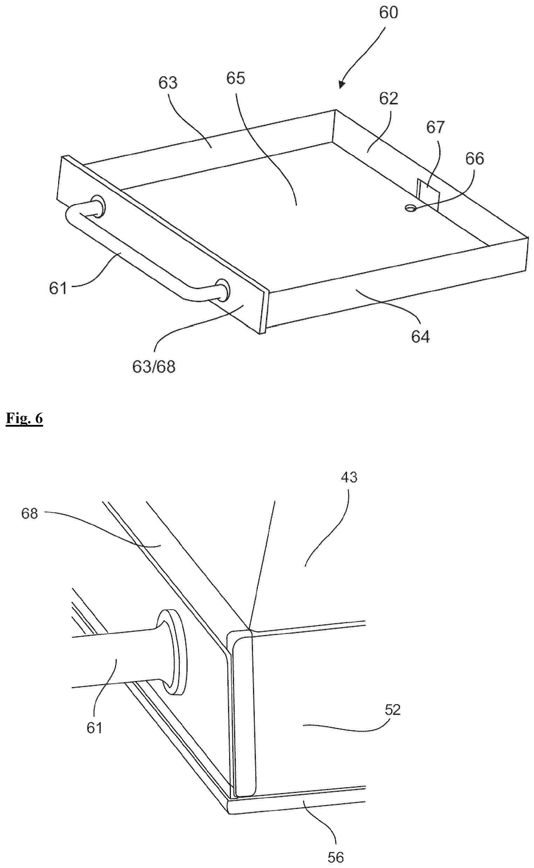

Figure 6 also shows a drawer 60 from front side. Drawer 60 comprises a drawer front 63, drawer side walls 64, a drawer back wall 62 and a drawer floor 65. Drawer handle 61 (not visible) and the light trap panel 68 mentioned above are arranged on drawer front 63. Drawer back 62 comprises a recess and a steel plate in the recess 67 positioned to engage with a magnet at the rear of the drawer cabinet 50, when drawer 60 is in closed position in the said drawer cabinet. Drawer floor 65 comprises a drawer aperture 66 for easy cleaning and support removal of an insert.

Figure 7 shows a detail of the light trap when the device 1 is assembled and the drawer 60 is closed in the drawer cabinet 50, showing how light trap panel 68 of the drawer nicely fits the enclosure of the drawer cabinet to form the light trap. Drawer light trap panel 68 does not cover stabilizing edge 56, so the device can lay flat on a table. Device components were obtained by 3D-printing; material was ABS. Components including mobile phone case were aligned and glued. A mobile phone, for example type Apple iPhone S2020, alternatively iPhone SE 2022, was positioned on in a suitable phone case connected to the upper surface 22 of holder 21 and used for image acquisition.

The components were produced by 3D printing. The device when glued was very stable despite low weight and thin walls (1.5 mm, total weight 341g without smartphone).

Figure 8 shows a diagram of the system comprising the imaging unit 20 of the imaging device connected with control unit 100 comprising one or more processors configured to conduct sets of instruction relating to one or more of.
(150) Prompting user for introducing or confirming metadata related to the sample, such as but not limited to identificator for seed sample, field, day and time, etc;
(151) Providing an image preview of the scene to be captured to the user by way of causing activating lighting sources for display of the illuminated scene on a display screen,
(152) Checking adequacy of imaging conditions by running a function of comparing imaging conditions measured by the imaging device with predefined thresholds;
(153) Prompting user for introducing new sample at same or different location;
(154) Prompting user that image capture/acquisition is completed;
(155) Computing discrete locations or a trajectory comprising discrete locations (also called an acquisition path) for image acquisition;
(141) Causing transmission of acquired image data, typically image together with related metadata, to one or more processors configured for image storage in an image repository (142), unit for image treatment and / or image analysis (80);
(142) Storing images and metadata (Image repository)
(143) Determining the geographical position of the imaging device at image acquisition.

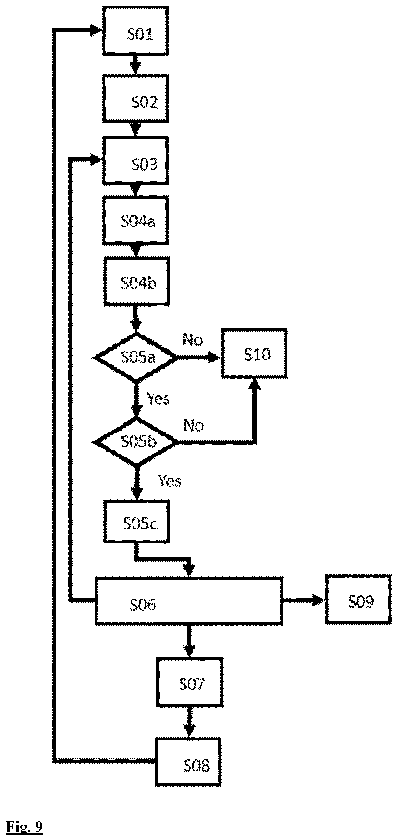

Figure 9 shows a flow diagram of a method for use of the image acquisition device for imaging of oilseed rape seeds collected in a field from oilseed rape pods.

The method is described using a smartphone comprising a camera for capturing images and an imaging control application comprising options and capabilities, such as capabilities for introducing metadata relating to the seed sample, supporting image acquisition, conducting image acquisition, transmitting acquired data for later use, but not limited thereto. The skilled person will appreciate that the camera and the hardware hosting the imaging control application may be separate devices connected with each other for transmission of data in one or both directions.

The method comprises:
501 - Preparing a seed sample: pods are collected in the field - the user removes the seeds from the pods, so that only the seeds are left.
S02 - Introducing the seed sample in the imaging device - the user opens the drawer and adds the seeds to the blue inlay/ seed collecting region and closes the drawer so the light trap and the magnetic system for maintaining the drawer in closed position is working.
S03 - Inputting metadata in the imaging control application - the user opens the imaging control application on the smartphone and enters the relevant metadata of the respective seed sample (e. g. country, field name, row & column of field, genotype, modality, batch number, replicate ID) in the corresponding application option. It is preferred that the imaging control application is configured so all metadata are transmitted and made available in a cloud database, wherein the metadata is linked to the respective image(s) for later use.

In an embodiment, ground truth values of moisture content and/or TKW measured according to known methods can be introduced in the imaging control application for each batch / sample, so the available ground truth values are saved in the metadata to the sample image and made available in the cloud database. This is of particular interest to generate a data set for training one or more models for computer implemented indirect measurement of moisture content and/or TKW based on color analysis of images of a seed sample.

S04 - Acquiring and recording image - the user opens an image recording feature in the imaging control application, wherein S04 comprises the following sub-steps:
S04a - Checking seed sample position - the flash-light of the cell phone is automatically switched on, so the user can check the position of the seeds in the positioning area, wherein the flashlight illuminates the seeds in the closed imaging device, that is in light-tight dark imaging chamber. If the seeds are not centered in the seed collecting region or stick on the rim, the device is shaken by the user to center the seeds. The tilted edges allow redistribution of the seeds in the positioning area. The use may reiterate the shaking until distribution is acceptable.

S04b - Recording image - The image is taken using the auto focus (centered) and flash-light of the cell phone camera. Auto focus and flashlight are enabled automatically for a smooth and easy handling.

S05 - Checking if image fulfills defined requirements for proper image analysis is preferably designed to conduct one or more of the following sub-steps:
S05a - Checking for enough seeds in the image (adequate seed count) - There should be minimum around 300 OSR seeds in the image to guarantee (good) statistically relevant results in computer implemented analysis of image seed color distribution. In an embodiment, the check assumes that 300 seeds of oilseed rape seeds as a monolayer roughly cover one quarter of the surface of the blue seed collecting region.

Based on this assumption, the user can evaluate the number of seeds by visual inspection of the surface covered with seeds or blue background. Alternatively or additionally, the imaging control application can comprise a feature for evaluation of covered surface of the seed collecting region and / or a seed counting algorithm configured to automatically counts the number of seeds in the image. It is preferred that, that the respective application feature is configured to cause a warning in the application in case there are not enough seeds in the image.

The skilled person will appreciate that covered surface may vary for different seeds sorts. In an embodiment, the introduction of the metadata related to the sample triggers selection of minimal surface to be covered based on historical averaged seed size data.

In most cases, the feature for checking number of seeds does not prevent the user from saving and / or transmitting the acquired image.

S5b - Checking image blurriness - In an embodiment, the application comprises a feature for automatic checking whether the image is in focus or blurry. For this purpose, the application uses an algorithm comparing the blurriness of the image with a threshold. For this purpose, the algorithm typically converts the color image to a gray scale image and a Laplacian function is used to calculate the blurriness coefficient. The application is configured to cause a warning if the image blurriness coefficient is below a certain threshold. The blurriness coefficient is saved in the image metadata and is available in the image data base.

Typically, blurriness check does not prevent the user from saving the image and / or transmitting the image the acquired image and related metadata.

S05c - Saving color image: The image is then saved and in case of a cloud connection transmitted to a cloud platform. Alternatively, the image can be stored in the control application until a cloud connection is enabled.

S06 - Acquiring technical images replicates - Typically, up to 5 images of the same seed sample are needed for sample relevant results of image analysis as each image only show the section of the seeds facing the camera. To acquire image replicates, the device is shaken by the user in between the different images so that different views and positions of the seeds are recorded. In other words, the steps S03 to S05c are repeated up to 5 times with shaking the device in between each new image. The tilted edges of the imaging device allow adequate redistribution of the seeds in the positioning area for new image.

In an embodiment, the imaging control application is configured to count the number of images acquired for a sample.

In an embodiment, the imaging control application is configured to cause prompting the user for shaking the device between two image acquisition until adequate number of images are acquired for the sample. In an embodiment, the imaging control application is configured to cause displaying information on the status of image acquisition, such as i of n images acquired, end of image acquisition, "ready to remove sample" or the like.

S07 - Removing sample - the user removes the seed sample from the drawer by pulling it out of the drawer cabinet. Usually, the seed sample can be easily poured off the drawer at the pouring corner. Occasionally, it is necessary to swipe the inlay to remove sticky seeds.

S08 - Image acquisition for biological replicates: In order to analyze the variability among one genotype/plot etc., several biological replicates, it is advantageous that further samples from the same genotype/plot etc. are collected (typically minimum 3 biological replicates) and repeat the steps S01 to S07 for each biological replicate.

Further, images of some of the biological replicates can be used for training the models for image analysis and images of other of the biological replicates can be used for testing/validating the models for image analysis.

S09 - Transmitting image data for/ image analysis

If an internet connection is available, the images are uploaded immediately to the cloud platform after the recording. The models (moisture content and TKW) are automatically run for each incoming image in the cloud. The model results for moisture content and TKW are sent back to the application and are displayed for each image.

If there is no internet connection available, the image analysis can be run offline directly in the application of the cell phone device. This might result in a reduced result accuracy as the calculation power and storage of the smartphone is restricted.

S10 - Generate warning - that image does not fulfill criteria for good quality analysis.

The method was developed and described in detail for oilseed rape seeds but the skilled person, will appreciate that the solution for image acquisition is applicable to any other seeds. The solution was developed to acquire images in a field but may be used at any other location.

The skilled person will also appreciate that the portable imaging device of the invention may be used for imaging of other pourable bulk material of small size requiring controlled imaging conditions, for example but not limited to imaging of convenience blend compositions, e.g. sand, cement or the like, for image-based composition analysis.

The solution of the invention was built and used to acquire images for computer-implemented color analysis and later correlation of seed moisture content and seed color. The skilled person will appreciate that the solution can be used to evaluate dryness, coating of pourable bulk material, or the like.

In this context, seed is not limiting the use of the presented solution and rather stand for generic description of the pourable bulk material of interest.

## Claims

1. An image acquisition device comprising:
A planar seed collecting region configured to receive a seed sample;
An opaque chamber delimited by a chamber floor comprising the seed collecting region and a chamber top connected by chamber side walls between the chamber floor and the chamber top;
An imaging unit comprising an image sensor positioned to acquire an image of a scene comprising at least part of a seed sample positioned in the seed collecting region;
Wherein the seed collecting region is delimited by walls at least partially tilted towards the planar seed collecting region with a tilting angle α to the planar the seed collecting region bigger than 120 degrees.

2. The image acquisition device according to claim 1, wherein the tilting angle α to the planar the seed collecting region is selected from 140 to 170 degrees.

3. The image acquisition device according to one of the preceding claims, wherein the imaging unit is positioned at the chamber top.

4. The image acquisition device according to one of the preceding claims, wherein the dimensions and angles of the opaque chamber are adjusted to optimal focus settings for the image sensor.

5. The image acquisition device according to any one of the preceding claims, further comprising a light-tight drawer placed on the chamber floor, wherein said drawer is delimited by drawer walls connected to a drawer floor building the seed collecting region.

6. The image acquisition device according to claim 4, further comprising an insert placed in the drawer, said insert comprising walls framing the planar seed collecting region, wherein said walls are tilted towards the planar seed collecting region with the tilting angle α to the planar the seed collecting region bigger than 120 degrees.

7. The image acquisition device according to any one of the claims 4 or 5, wherein two of the tilted walls of the insert are configured to build a pouring corner.

8. The image acquisition device according to any one of the preceding claims, wherein the seed collecting region is blue.

9. The image acquisition device according to any one of the preceding claims, wherein the seed collecting region has a rough or structured surface.

10. The image acquisition device according to any one of the preceding claims, wherein the opaque chamber comprises assembled elements made of plastic.

11. The image acquisition device according to any one of the preceding claims, wherein thicknesses of the walls and / or seed collecting region are from 0.5 to 3 mm thin.

12. A system comprising at least one image acquisition device according to one of the claims 1 to 11 and a control unit comprising one or more processors configured for conducting the image acquisition by the image acquisition device.

13. The system according to claim 12, wherein the one or more processors are configured for one or more of:
Prompting user for introducing or confirming metadata related to seed sample;
Activating the light source and / or a function of test imaging for checking positioning of the seeds on the seed imaging surface by user;
Automatically checking predefined imaging conditions are fulfilled for image acquisition, prompting user for triggering image acquisition or correcting imaging conditions;
Causing prompting user that image acquisition is completed; and / or
Causing prompting user that the imaging device is ready for loading of a new sample.

14. A method comprising:
Placing a seed sample on a seed collecting region of an imaging device according to one of the claims 1 to 11;
Causing the imaging unit acquiring the one or more images of a scene comprising at least part of a seed sample positioned in the seed collecting region, preferably of a scene comprising the whole seeds collecting region.

15. The method according to claim 14, wherein the seed sample comprises a plurality of seeds.

16. The method according to any one of the preceding claims, further comprising one or more of:
Prompting user for introducing seed sample and optionally metadata related to the seed sample;
Causing the imaging device generating an image preview and / or a test image of the seed collecting region by way of activating the lighting source and / or a function of test imaging;
Causing checking adequacy of imaging conditions by displaying image preview and / or test image to the user, alternatively by running a function of comparing imaging conditions with predefined thresholds; and / or
Prompting user that image acquisition is completed;
Causing transmission of acquired image data to one or more processors configured for image storage, image treatment and / or image analysis.
